(19) Europäisches Patentamt
European Patent Office
Office européen des brevets

(11) Veröffentlichungsnummer: 0 004 951
B1

(12) # EUROPÄISCHE PATENTSCHRIFT

(45) Veröffentlichungstag der Patentschrift: 13.01.82

(21) Anmeldenummer: 79101134.9

(22) Anmeldetag: 12.04.79

(51) Int. Cl.$^3$: C 07 D 491/10,
A 61 K 31/445
//(C07D491/10, 221/00,
307/00)

(54) **N-(3-Benzoyl-propyl)-spiro-(dihydrobenzofuranpiperidine und -pyrrolidine) und Derivate, Verfahren zu ihrer Herstellung und ihre Verwendung für Arzneimittel.**

(30) Priorität: 14.04.78 US 896584

(43) Veröffentlichungstag der Anmeldung:
31.10.79 Patentblatt 79/22

(45) Bekanntmachung des Hinweises auf die Patenterteilung:
13.01.82 Patentblatt 82/2

(84) Benannte Vertragsstaaten:
DE FR GB IT

(56) Entgegenhaltungen:
DE - A - 2 458 176
DE - A - 2 458 177
US - A - 2 500 714
US - A - 3 686 186

JOURNAL OF MEDICINAL CHEMISTRY, Band 19, Nr. 11, 1976
Washington, USA
V. J. BAUER et al. "Synthesis of spiro [isobenzofuran-1(3H),4'piperidines] as potential central nervous system agents 1."
Seiten 1315 bis 1324

(73) Patentinhaber: HOECHST AKTIENGESELLSCHAFT
Postfach 80 03 20
D-6230 Frankfurt/Main 80 (DE)

(72) Erfinder: Effland, Richard Charles
Rolling Hills Road 544
Brigdewater, N.J. (US)
Erfinder: Strupczewski, Joseph Thomas
Garden Lane 8
Flemington, N.J. (US)
Erfinder: Gardner, Beth Ann
Calcaterra Drive 7038
San Jose, California (US)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 951 B1

N-[3-Benzoyl-propyl]-spiro-[dihydrobenzofuranpiperidine und -pyrrolidine] und Derivate, Verfahren zu ihrer Herstellung und ihre Verwendung für Arzneimittel

Gegenstand der vorliegenden Erfindung sind neue N-[3-(4-Fluorbenzoyl)-propyl]-spiro-[dihydrobenzofuranpiperidine und -pyrrolidine] sowie pharmazeutisch unbedenkliche Säureadditionssalze davon, Verfahren zu deren Herstellung und pharmazeutische Präparate, die diese Verbindungen als Hauptwirkstoffe enthalten.

Die Verbindungen der Erfindung haben die Formel

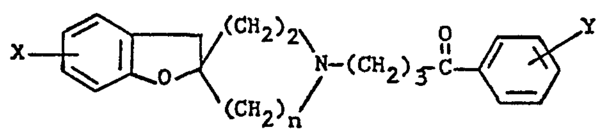

I

worin

X Wasserstoff, Nitro, Amino, Halogen, Methoxy oder Hydroxy,
Y Nitro, Amino, Halogen, Methoxy oder Hydroxy und
n 1 oder 2 bedeuten.

Als geeignete Säuren für die Herstellung der pharmazeutisch unbedenklichen sauren Salze der vorliegenden Erfindung seien organische Säuren wie beispielsweise Salzsäure, Bromwasserstoffsäure, Schwefelsäure, Salpetersäure, Phosphorsäure, und Perchlorsäure sowie organische Säuren wie beispielsweise Weinsäure, Zitronensäure, Essigsäure, Bernsteinsäure, Maleinsäure und Fumarsäure erwähnt.

Unter den Verbindungen der Formel I sind jene bevorzugt, in denen X Wasserstoff, Nitro oder Chlor und Y Fluor bedeuten.

Die erfindingsgemäßen Verbindungen sind noch nicht beschrieben worden. Es wurden bisher von W. J. Houlihan et al. im US Patent 3 686 186 Spiro-[phthalanpiperidine] der Formel

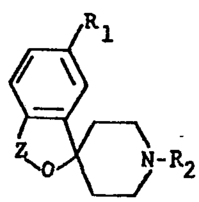

beschrieben,
worin

$R_1$ Wasserstoff, niederes Alkyl, niederes Alkoxy, Halogen oder Trifluormethyl,
$R_2$ Wasserstoff oder Benzyl und
Z $-CH_2$ oder $-CO-$

bedeuten, ferner von Y.Inubushi et al. die natürliche Verbindung der Formel

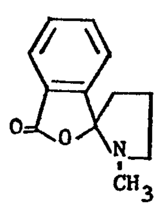

[Chem. and Pharm. Bull (Japan), *12,* 749 (1964)] sowie substituierte 1,3-Dihydrospiro-(isobenzofurane) der Formel

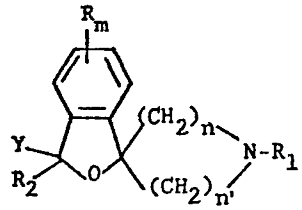

worin

R Wasserstoff, Alkyl, Alkoxy, Trifluormethyl, Halogen, Hydroxy oder Methylendioxy,

$R_1$ Wasserstoff, Alkyl, Cycloalkylalkyl, Alkenyl, Phenylalkyl, Diphenylalkyl, Diphenylmethoxyalkyl, Alkanoyl, Phenylalkanoyl, Benzoyl, Benzoylalkyl, Phenylhydroxyalkyl, Alkoxycarbonyl, Phenyloxycarbonyl oder Cycloalkylcarbonyl,
$R_2$ Alkyl oder Phenyl,
Y Wasserstoff, Alkyl, Alkoxy, Hydroxy oder Phenyl,
und m, n und n' Zahlen von 1 bis 3 bedeuten
und schließlich von Victor J. Bauer und Raymond W. Kosley, Jr. in den US Patenten 3 959 475 und 3 962 259, 1,3-Dihydrospiro-(isobenzofurane) der Formel

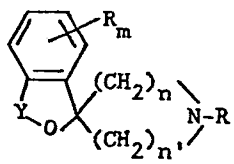

worin
R Wasserstoff, Alkyl, Alkoxy, Trifluormethyl, Halogen, Hydroxy oder Methylendioxy,
$R_1$ Alkyl, Cycloalkylalkyl, Phenylalkyl, Diphenylalkyl, Diphenylmethoxyalkyl, Alkanoyl, Phenylalkanoyl, Benzoylalkyl, Phenylhydroxyalkyl oder Cycloalkylcarbonyl,
Y $CH_2$ oder CO,
m 1 oder 2 und
n und n' ganze Zahlen von 1 bis 3
bedeuten.

Die Verbindungen der vorliegenden Erfindung werden hergestellt, indem man

a) ein N-unsubstituiertes Spiro-[dihydrobenzofuranpiperidin oder -pyrrolidin] der Formel

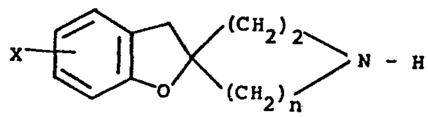

II

worin X und n die in Formel I genannte Bedeutung haben mit einem $\gamma$-Chlorbutyrophenonäthylenglykolketal der Formel

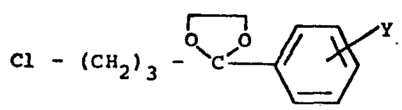

III

worin Y die in Formel I genannte Bedeutung hat, in einem Lösungsmittel, vorzugsweise Dimethylformamid und Butylalkohol, zu einer Verbindung der Formel

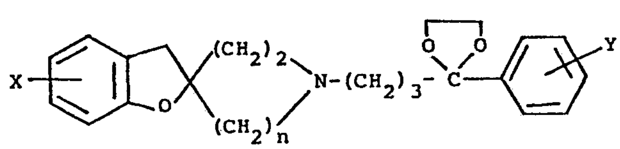

IV

bei einer Reaktionstemperatur von ca. 25°C bis zur Rückflußtemperatur des Reaktionsgemisches, gegebenenfalls in Anwesenheit eine Säurefängers wie Kaliumcarbonat und eines Reaktionsauslösers im Reaktionsgemisch umsetzt.

b) das erhaltene Ketal, vorzugsweise mit einer starken Säure wie 3n Salzsäure, in einem Lösungsmittel bei Zimmertemperatur zur entsprechenden N-[3-Benzoylpropyl]-Verbindung der Formel I hydrolysiert und

c) gegebenenfalls eine Verbindung der Formel I, worin X und/oder Y Methoxy bedeuten, nach geeigneten bekannten Methoden, vorzugsweise mit 43%iger Bromwasserstoff säure, unter Rückflußbedingungen, zur entsprechenden Verbindung der Formel I, worin X und/oder Y Hydroxy bedeuten, demethyliert.

Die in Verfahren a) eingesetzte N-unsubstituierte Vorstufe der Formel II kann wie folgt hergestellt werden:

1) Ein 2-Fluorbenzylchlorid oder -bromid der Formel

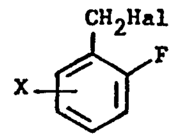

V

worin X die Formel I angegebene Bedeutung hat und Hal Chlor oder Brom bedeutet, wird vorzugsweise unter Verwendung von Äther als Lösungsmittel und von einem Jodkristall als Reaktionsauslöser zu seinem Grignard-Reagenz umgesetzt. Das Grignard-Reagenz wird dann mit einem Cycloazalkanon der Formel

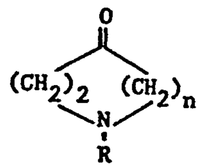

VI

worin n die in Formel I angegebene Bedeutung hat und R Alkyl, Alkenyl, Cycloalkylalkyl oder Phenylalkyl sein kann, zu einer Verbindung der Formel

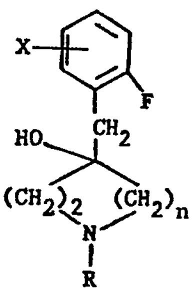

VII

umgesetzt.

Letztere wird mit einer nicht-nukleophilen Base in Gegenwart eines Lösungsmittels bei einer Temperatur von 25°C bis zur Rückflußtemperatur des Lösungsmittels zu einer Verbindung der Formel

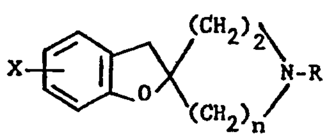

(II)

worin R die oben angegebene Bedeutung hat, umgesetzt. In einer bevorzugten Ausführungsform werden Natriumhydrid als Base und Dimethylformamid und Benzol als Lösungsmittelgemisch bei Rückflußtemperatur eingesetzt.

Eine Verbindung der Formel II, worin R Benzyl bedeutet, kann nach geeigneten Methoden zur entsprechenden N-unsubstituierten Vorstufe, vorzugsweise unter Verwendung eines Palladium/Kohlenstoff-Katalysators, hydriert werden.

Eine Verbindung der Formel II kann weiterhin mit einem Chlorameisensäureester wie beispielsweise ein Chlorameisensäurealkyl- oder -phenylester bei einer Temperatur von 25—125°C in einem Lösungsmittel wie Toluol oder Benzol zur entsprechenden N-Alkoxycarbonyl- oder N-Phenoxycarbonyl-Verbindung umgesetzt werden.

Die erhaltene N-Alkoxycarbonyl- oder N-Phenoxycarbonyl-Verbindung wird dann unter Rückflüßbedingungen mit einer Base wie Natrium- oder Kaliumhydroxid in einem Lösungsmittel wie Wasser oder Äthanol oder mit einer Säure wie Bromwasserstoffsäure in Essigsäure zur entsprechenden N-unsubstituierten Vorstufe umgesetzt.

Eine Vorstufe, worin X Wasserstoff bedeutet kann ebenfalls in bekannter Weise, z.B. in einem Lösungsmittel wie Pyridin oder Chloroform mit einem Alkanoylhalogenid oder -anhydrid zur entsprechenden Verbindung der Formel II, worin R Alkanoyl und X Wasserstoff bedeuten, gegebenenfalls unter Verwendung eines Säurefängers wie Natriumbicarbonat, Kaliumcarbonat oder Triäthylamin umgesetzt werden. Die Reactionstemperatur kann von ca. 0°C bis zur Rückflußtemperatur des Lösungsmittels variieren, wobei vorzugsweise unter Rückflußbedingungen gearbeitet wird. Die Verbindung worin R Alkanoyl bedeutet, kann mit einem Gemisch aus einer konzentrierten Salpetersäure in Eisessig

zur entsprechenden Verbindung der Formel I, die in 5-Stellung durch Nitro substituiert ist, bei einer Temperatur von 15 bis 150°C, vorzugsweise 100°C, umgesetzt werden.

Schließlich kann eine N-unsubstituierte Verbindung der Formel I, worin X $NO_2$ bedeutet, zur entsprechenden Verbindung, worin X Amino bedeutet, katalytisch reduziert werden, z.B. durch Hydrierung unter erhöhtem Druck unter Verwendung eines Raney-Nickel-Katalysators.

Die Verbindungen der Formel I und IV sind wertvolle Analgetica aufgrund ihrer Fähigkeit, Schmerzen von Säugetieren zu lindern. Diese analgetische Wirksamkeit läßt sich mit dem "Phenyl-2-chinon-Strecktest" an Mäusen, einem Standardtest für Analgetica, nachweisen (Proc. Soc. Exptl. Biol. Med., *95,* 729 (1957)). In nachfolgender Tabelle ist die schmerzstillende Wirkung nach subkutaner Verabreichung verschiedener Dosen typischer Vertreter der Verbindungen der vorliegenden Erfindung als Prozentsalz der Fälle aufgeführt, in denen das "Sich krümmen" der Versuchstiere infolge von Schmerz verhindert wird.

| Verbindung | Dosis mg/kg | Schmerzstillung (in % der Tiere) |
|---|---|---|
| 2,3-Dihydro-1′-[3-(4-fluorbenzoyl)-propyl]-5-nitrospiro-[benzofuran-2,4′-piperidin]-hydrochlorid | 0,4 | 50 |
| 2,3-Dihydro-1′-[3-(4-fluorbenzoyl)-propyl]-6-chlorspiro-[benzofuran-2,4′-piperidin]-hydrochlorid | 1,8 | 50 |
| 2,3-Dihydro-1′-[3-(4-fluorbenzoyl)-propyl]-5-chlorospiro-[benzofuran-2,4′-piperidin]-hydrochlorid | 1,7 | 50 |
| 2,3-Dihydro-1′-[3-(4-fluorbenzoyl)-propyl]-spiro-[Benzofuran-2,4′-piperidin]-hydrochlorid | 10<br>6,0* | 93<br>50 |

* orale Dosis

Im Vergleich dazu erzielt man mit den bekannten Analgetica Aspirin und Propoxyphen, mit einer Dosis von 60 mg/kg bzw. 28 mg/kg eine 34%ige bzw. 50%ige Schmerzstillung. Daraus wird ersichtlich, daß die Verbindungen gemäß der Erfindung geeignete schmerzlindernde Mittel für Säugetiere sind, wenn sie in Mengen von ca. 0,05 — ca. 100 mg/kg Körpergewicht pro Tag verabreicht werden.

Die Verbindungen der Formel I und IV eignen sich ebenfalls als Tranquilizer aufgrund ihrer Fähigkeit, beruhigend aus das zentrale Nervensystem von Säugetieren einzuwirken. Diese Fähigkeit läßt sich mit dem "Sidman Avoidance-Test" (Science, 118, 157—8, (1953)), einem Standardtest für Tranquilizer, wonach 2,3-Dihydro-1′-[3-(4-fluorbenzoyl)-propyl]-spiro-[benzofuran-2,4′-piperidin]-hydrochlorid in einer intraperitonalen Dosierung von 11,1 mg/kg eine 50%ige Herabsetzung des erlernten Vermeidens von Elektroschocks bei Ratten bewirkt, nachweisen. Bei Verabreichung in Tagesdosen von 0,05 — ca. 100 mg/kg eignen sich die Verbindungen der Erfindung als Tranquilizer.

Weitere erfindungsgemäße Verbindungen sind unter anderem:

2,3-Dihydro-5-amino-1′-[3-(4-fluorbenzoyl)-propyl]-spiro-[benzofuran-2,4′-piperidin];
2,3-Dihydro-1′-[3-(4-fluorbenzoyl)-propyl]-5-methoxyspiro-[benzofuran-2,4′-piperidin];
2,3-Dihydro-1′-[3-(4-fluorbenzoyl)-propyl]-5-fluorspiro-[benzofuran-2,4′-piperidin];
2,3-Dihydro-1′-[3-(4-fluorbenzoyl)-propyl]-6-methoxyspiro-[benzofuran-2,4′-pyrrolidin];
2,3-Dihydro-1′-[3-(4-fluorbenzoyl)-propyl]-6-bromspiro-[benzofuran-2,4′-pyrrolidin];
2,3-Dihydro-1′-[3-(4-nitrobenzoyl)-propyl]-spiro-[benzofuran-2,4′-piperidin];
2,3-Dihydro-1′-[3-(4-methoxybenzoyl)-propyl]-spiro-[benzofuran-2,4′-piperidin];
2,3-Dihydro-1′-[3-(2-brombenzoyl)-propyl]-spiro-[benzofuran-2,4′-piperidin];
2,3-Dihydro-1′-[3-(4-aminobenzoyl)-propyl]-spiro-[benzofuran-2,4′-piperidin];
2,3-Dihydro-1′-[3-(2-nitrobenzoyl)-propyl]-spiro-[benzofuran-2,3′- pyrrolidin];
2,3-Dihydro-1′-[3-(3-methoxybenzoyl)-propyl]-spiro-[benzofuran-2,3′-pyrrolidin];
2,3-Dihydro-1′-[3-(3-brombenzoyl)-propyl]-spiro-[benzofuran-2,3′-pyrrolidin];
2,3-Dihydro-1′-[3-(4-brombenzoyl)-propyl]-spiro-[benzofuran-2,3′-pyrrolidin];
2,3-Dihydro-1′-[3-(4-aminobenzoyl)-propyl]-spiro-[benzofuran-2,3′-pyrrolidin];
2,3-Dihydro-1′-[3-(4-hydroxybenzoyl)-propyl]-spiro-[benzofuran-2,4′-piperidin];
2,3-Dihydro-1′-[3-(2-hydroxybenzoyl)-propyl]-spiro-[benzofuran-2,3′-pyrrolidin];
2,3-Dihydro-5-hydroxy-1′-[3-(4-hydroxybenzoyl)-propyl]-spiro-[benzofuran-2,4′-piperidin];
2,3-Dihydro-5-methoxy-1′-[3-(2-hydroxybenzoyl)-propyl]-spiro-[benzofuran-2,4′-piperidin];

2,3-Dihydro-4-amino-1'-[3-(4-fluorbenzoyl)-propyl]-spiro-[benzofuran-2,4'-piperidin];
2,3-Dihydro-1'-[3-(4-fluorbenzoyl)-propyl]-4-methoxyspiro-[benzofuran-2,3'-pyrrolidin];
2,3-Dihydro-1'-[3-(4-fluorbenzoyl)-propyl]-7-fluorspiro-[benzofuran-2,4'-piperidin];
2,3-Dihydro-1'-[3-(4-fluorbenzoyl)-propyl]-7-nitrospiro-[benzofuran-2,3'-pyrrolidin]; und
2,3-Dihydro-7-chlor-1'-[3-(2-nitrobenzoyl)-propyl]-spiro-[benzofuran-2,4'-piperidin].

Wirksame Mengen der erfindungsgemäßen Verbindungen können einem Patienten nach den verschiedensten Methoden, beispielsweise oral in Kapsel- oder Tablettenform, parenteral in Form von sterilen Lösungen oder Suspensionen und in einigen Fällen intravenös in Form von sterilen Lösungen, verabreicht werden. Die Endprodukte selbst sind zwar als frei Base wirksam, doch kann man sie aus Gründen der Stabilität, einer besseren Kristallisierbarkeit, einer höheren Löslichkeit usw. in Form ihrer pharmazeutisch unbedenklichen Additionssalze formulieren und verabreichen.

Die erfindungsgemäßen Wirkstoff können oral verabreicht werden, z.B. mit einem inerten Verdünnungsmittel oder mit einem eßbaren Träger, in Gelatinekapseln eingeschlossen werden oder zu Tabletten verpreßt werden. Zur peroralen therapeutischen Verabreichung können die erfindungsgemäßen Wirkstoffe in Trägerstoffe eingearbeitet werden und in Form von Tabletten, Pastillen, Kapseln, Elixieren, Suspensionen, Sirupen, Oblaten, Kaugummi und dergleichen verabreicht werden. Diese Präparate sollten wenigstens 0,5% Wirkstoff enthalten, doch läßt sich dies in Abhängigkeit von der jeweiligen Form variieren. Zweckmäßigerweise beträgt der Wirkstoffgehalt 4 — ca. 70% des Einheitsgewichts. Die Wirkstoffmenge sollte so bemessen sein, daß sich eine entsprechende Dosis ergibt. Bevorzugte Mittel und Präparate gemäß vorliegender Erfindung werden so zubereitet, daß eine perorale Dosiereinheitsform 1,0—300 mg Wirkstoff enthält.

Die Tabletten, Pillen, Kapseln, Pastillen usw. können ferner folgende Bestandteile enthalten: ein Bindemittel wie mikrokristalline Cellulose, Traganthgummi oder Gelatine: Vehikel wie Stärke oder Milchzucker; Zerfallmittel wie Alginsäure, oder Maisstärke usw; Schmiermittel wie Magnesiumstearat; Gleitmittel wie kolloides Siliciumdioxid; einen Süßstoff wie Rohrzucker oder Saccharin oder einen Aromastoff wie Pfefferminz, Methylsalicylat oder Orangenaroma. Ist die Dosiereinheitsform eine Kapsel, so kann diese neben obigen Stoffen eine flüssigen Träger wie pflanzliche Öle enthalten. Andere Dosiereinheitsformen können weitere Stoffe enthalten, die die physikalische Form der Dosiereinheit ändern, z.B. Überzüge. So können Tabletten und Pillen mit Zucker, Schellack oder anderen erst im Darm löslichen Überzügen versehen werden. Ein Sirup kann neben den Wirkstoffen Rohrzucker als Süßstoff und einige Konservierungsmittel, Farbstoffe und Aroma stoffe enthalten. Die bei der Zubereitung dieser verschiedenen Mittel verwendeten Stoffe müssen pharmazeutisch rein und nicht-toxisch in den angewendeten Mengen sein.

Zur parenteralen therapeutischen Verabreichung können die erfindungsgemäßen Verbindungen in eine Lösung oder Suspension eingearbeitet werden. Die resultierenden Präparate sollten wenigstens 0,1% Wirkstoff enthalten, doch läßt sich dies zwischen 0,5 und ca. 30%, bezogen auf ihr Gewicht, variieren. Die Wirkstoffmenge in diesen Mitteln sollte so bemessen sein, daß eine entsprechende Dosis sich ergibt. Bevorzugte Mittel und Präparate gemäß vorliegender Erfindung werden so hergestellt, daß eine parenterale Dosiereinheit 0,5—100 mg Wirkstoff enthält.

Die Lösungen oder Suspensionen können ferner folgende Bestandteile enthalten: ein steriles Verdünnungsmittel wie Wasser pro injectione, physikalische Kochsalzlösung, nichtflüchtige Öle, Polyäthylenglykole, Glycerin, Propylenglykol oder andere synthetische Lösungsmittel; antibakteriell wirksame Mittel wie Benzylalkohol; Antioxidantien wie Ascorbinsäure oder Natriumbisulfit; Chelatbildner wie Äthylendiamintetraessigsäure; Puffersubstanzen wie Acetate, Citrate oder Phosphate und Mittel zur Einstellung des osmotischen Druckes wie Kochsalz oder Dextrose. Die parenterale Zubereitung kann in Anpullen, (Einwegspritzen) oder Mehrfachdosisspritzen aus Glas oder Plastik eingeschmolzen werden.

In den nachfolgenden Beispielen wird die Erfindung näher erläutert:

## Beispiel 1

a) Ein Gemisch aus 2,7 g Magnesiumspänen in 25 ml Äther, das einen Jodkristall enthält, wird mit einigen Millilitern 2-Fluorbenzylchlorid (in einer Lösung aus 14,5 g 2-Fluorbenzylchlorid in 75 ml Äther) versetzt. Die Reaktion wird durch vorsichtiges Erwärmen mit heißer Luft in Gang gesetzt. Nach beginnender Reaktion tropft man die restliche 2-Fluorbenzylchloridlösung zu, wobei das Reaktionsgemisch auf Rückflußtemperatur erhitzt wird Nach beendeter Zugabe rührt man das Reaktionsgemisch unter Rückfluß weitere 15 Minuten und tropft dann unter heftigem Rühren eine Lösung aus 25,0 g N-Benzyl-4-piperidon in 75 ml Äther zu. Die erhaltene Suspension wird dann bei Zimmertemperatur 2 Stunden gerührt und filtriert. Der Filterkuchen wird sorgfältig mit Äther gewaschen und mit einer Ammoniumchloridlösung unter Rühren hydrolysiert. Die wässrige Mischung wird mit Äther extrahiert, die vereinigten Ätherextrakte werden getrocknet, und der Äther wird entfernt, wobei ein gelblich-grünes Öl zurückbleibt, das destillativ gereinigt wird. Als Rückstand erhält man dabei ein gelbes Öl, das nach einigem Stehen zu dem hellgelben festem 1-Benzyl-4-(2-fluorbenzyl)-4-piperidinol erstarrt.

b) Eine Suspension aus 1,4 g 50%igen Natriumhydrid in 50 ml Dimethylformamid und 10 ml Benzol wird unter Rühren mit einer Lösung aus 6,9 g 1-Benzyl-4-(2-fluorbenzyl)-4-piperidinol in 25 ml

Dimethylformamid und 10 ml Benzol versetzt. Die Mischung wird dann bei 110—120°C 5 Stunden in Anwesenheit eines Luftkühlers erhitzt, um das Benzol verdampfen zu lassen. Dann läßt man die Mischung auf Zimmertemperatur abkühlen, gibt das abgekülte Gemisch auf Eis, verdünnt es mit Wasser und extrahiert es mit Äther. Die Ätherphase wird nacheinander mit Wasser und gesättigter wässriger Kochsalzlösung gewaschen und getrocknet. Der Äther wird aus der getrockneten Lösung entfernt, wobei ein Öl zurückbleibt, das beim Stehenlassen zu einem hellgelben Feststoff erstarrt. Der Feststoff wird in Äther gelöst und die Lösung mit ätherischer Salzsäure behandelt, wobei ein Salz in Form eines weißen Feststoffes anfällt, der in Isopropylalkohol zu weißen 2,3-Dihydro-1′-benzyl-spiro-[benzofuran-2,4′-piperidin]-hydrochloridkristallen umkristallisiert wird. Schmelzpunkt: 246—247°C.

| Analyse: | | | |
|---|---|---|---|
| Berechnet für $C_{19}H_{21}NO \cdot HCl$: | 72,24% C; | 7,03% H; | 4,44% N |
| Gefunden: | 71,95% C; | 7,06% H; | 4,34% N |

c) Eine Lösung aus 5,3 g 2,3-Dihydro-1′-benzylspiro-[benzofuran-2,4′-piperidin], die freie Base aus der Verbindung in b), wird in 250 ml Isopropylalkohol unter Verwendung eines Schüttelapparates (nach Paar) unter 3,5 atü bei 65—70°C in Gegenwart von 1 g eines 10%igen Palladium/Kohlenstoffkatalysators bis zur vollständigen Wasserstoffaufnahme hydriert. Dann läßt man die Lösung auf Zimmertemperatur abkühlen, filtriert sie und dampft sie zur Trockne ein, wobei ein weißer Feststoff zurückbleibt, der in einer Benzol/Äthermischung gelöst wird. Die erhaltene Lösung wird durch Celite® filtriert und dann zu einem weißen Feststoff eingedampft, der mit Äther verrieben wird, wobei 2,3-Dihydrospiro-[benzofuran-2,4′-piperidin] anfällt. Schmelzpunkt: 56—58,5°C.

| Analyse: | | | |
|---|---|---|---|
| Berechnet für $C_{12}H_{15}NO$: | 76,14% C; | 8,00% H; | 7,40% N |
| Gefunden: | 76,05% C; | 8,08% H; | 7,27% N. |

d) Eine Lösung aus 5,0 g 2,3-Dihydrospiro-[benzofuran-2,4′-piperidin], 8,3 g Kaliumcarbonat, 5,0 g Kaliumjodid und 7,1 g $\gamma$-Chloro-p-fluorbutyrophenonäthylenglykolketal in 75 ml Dimethylformamid wird 18 Stunden bei 90°C gerührt. Dann läßt man das Reaktionsgemisch auf Zimmertemperatur abkühlen und dampft das überschüssige Lösungsmittel ab, wobei ein hellohfarbener halb-fester Stoff anfällt. Dieser Rückstand wird in Benzol gelöst und nacheinander mit Wasser und gesättigter Kochsalzlösung gewaschen. Dann wird das Benzol abgedampft, wobei ein dunkles Öl zurückbleibt, das in einer Lösung aus 75 ml Methylalkohol und 40 ml 3n-Salzsäure gelöst wird. Die erhaltene Lösung wird 24 Stunden bei Zimmertemperatur gerührt.

Dann wird die Reaktionslösung mit 2n-Natronlauge basisch gemacht, zweimal mit Chloroform extrahiert und getrocknet. Das Chloroform wird abgedampft, wobei ein Öl zurückbleibt, das in Äther gelöst wird und dort in sein Chlorwasserstoffsalz, einem weißen Niederschlag, überführt wird. Das Salz wird dreimal in Äthylalkohol umkristallisiert, wobei 2,3-Dihydro-1′-[3-(4-fluorbenzoyl)-propyl]-spiro-[benzofuran-2,4′-piperidin]-hydrochlorid anfällt. Schmelzpunkt: 232—235°C.

| Analyse: | | | | | |
|---|---|---|---|---|---|
| Berechnet für $C_{22}H_{24}FNO_2 \cdot HCl$ | 67,77% C; | 6,46% H; | 3,59% N; | 9,09% Cl; | 4,87% F |
| Gefunden: | 67,98% C; | 6,65% H; | 3,68% N; | 9,20% Cl; | 5,10% F. |

## Beispiel 2

a) Eine Suspension aus 3,4 g Magnesiumspänen in 50 ml Äther wird mit einem Jodkristall und einigen Millilitern einer Lösung aus 20,6 g 2-Fluorbenzylchlorid in 100 ml Äther versetzt. Dann wird die Reaktion mit einem Heißluftgebläse ausgelöst und die restliche 2-Fluorbenzylchloridlösung wird unter Erhitzen auf Rückflußtemperatur zugetropft. Nach beendeter Zugabe beläßt man die Reaktionsmischung eine weitere Stunde unter Rühren bei Rückflußtemperatur und gibt dann nacheinander 100 ml Äther und unter heftigem Rühren eine Lösung aus 25,0 g N-Benzyl-3-pyrrolidon in 100 ml Äther hinzu. Nach beendeter Zugabe wird die erhaltene Suspension 2 Stunden unter Rückfluß erhitzt, bei Zimmertemperatur 16 Stunden gerührt und filtriert. Der Filterkuchen wird sorgfältig mit Äther gewaschen und dann unter Rühren in einer Eis-Ammoniumchloridlösung hydrolysiert. Die wäßrige Lösung wird dreimal mit Äther gewaschen, und die vereinigten Ätherextrakte werden nacheinander mit Wasser und Kochsalzlösung gewaschen und getrocknet. Das Lösungsmittel wird entfernt und der Rückstand be 135—140°C unter einem Druck von 0,1 mm Hg destilliert, wobei ein zähflüssiges gelbes Öl zurückbleibt, das nach längerem Stehen erstarrt. Der Feststoff wird zweimal aus einem Äthanol/Wasser-Gemisch umkristallisiert, wobei man 1-Benzyl-3-(2-fluorbenzyl)-3-pyrrolidinol erhält. Schmelzpunkt: 75—77°C.

b) Zu einer Suspension aus 4,7 g Natriumhydrid in 125 ml Benzol und 125 ml Dimethylformamid tropft man bei 90°C unter Rühren 38,1 g 1-Benzyl-3-(2-fluorbenzyl)-3-pyrrolidinol als Lösung in 125 ml Benzol und 125 ml Dimethylformamid. Nach beendeter Zugabe wird die Reaktionsmischung 120 Stunden bei 90°C weitergerührt. Dan läßt man die Reaktionsmischung auf Zimmertemperatur

abkühlen, und gibt sie auf 1 l Eiswasser. Das Zweiphasengemisch wird mit Äther extrahiert, und die vereinigten Ätherextrakte werden getrocknet und zu einem dunklen Öl eingedampft. Das Öl wird bei 170—175°C unter einem Druck von 0,1 mm Hg destilliert, wobei 24 g eines hellgelben Öls anfallen. Das Öl erstarrt bei längerem Stehenlassen. Der erhaltene Feststoff wird zweimal aus Isopropylalkohol umkristallisiert, wobei man 2,3-Dihydro-1'-benzylspiro-[benzofuran-2,3'-pyrrolidin] erhält. Schmelzpunkt: 43—45°C.

Analyse:

| | | | |
|---|---|---|---|
| Berechnet für $C_{18}H_{19}NO$: | 81,47% C; | 7,22% H; | 5,28% N; |
| Gefunden: | 81,59% C; | 7,39% H; | 5,16% N. |

c) Eine Lösung aus 21,4 g 2,3-Dihydro-1'-benzylspiro-[benzofuran-2,3'-pyrrolidin] und 200 ml Isopropylalkohol wird über 2,0 g eines 10%igen Palladium/Kohlenstoff-Katalysators unter einem Druck von 3,5 atü bei 50°C bis zur vollständingen Wasserstoffaufnahme hydriert. Dann wird die Reaktionsmischung filtriert und unter vermindertem Druck zu einem gelben Öl eingeengt. Das Öl wird in Äther gelöst und dort in sein Chlorwasserstoffsalz überführt. Dieses Salz wird dann zweimal aus einer Äthylalkohol-Äthermischung zu 2,3-Dihydrospiro-[benzofuran-2,3'-pyrrolidin]-hydrochlorid umkristallisiert. Schmelzpunkt: 174—178°C.

Analyse:

| | | | | |
|---|---|---|---|---|
| Berechnet für $C_{11}H_{13}NO \cdot HCl$: | 62,41% C; | 6,67% H; | 6,62% N; | 16,75% Cl; |
| Gefunden: | 62,17% C; | 6,72% H; | 6,58% N; | 16,60% Cl. |

d) Eine Lösung aus 7,2 g 2,3-Dihydrospiro-[benzofuran-2,4'-pyrrolidin], die freie Base aus c), 14 g Kaliumcarbonat, einigen Kaliumjodidkörnern und 11 g $\gamma$-Chlor-p-fluorbutyrophenonäthylenglykolketal in 100 ml Dimethylformamid wird 2,4 Stunden bei 100°C erhitzt. Dann läßt man die Reaktionslösung auf Zimmertemperatur abkühlen, filtriert sie und dampft sie zur Trockne zu einem dunklen Öl ein. Das Öl wird in 100 ml Methylalkohol und 50 ml 3n-Salzsäure gelöst, und die erhaltene Lösung wird 18 Stunden gerührt. Die Lösung wird dann mit 3n-Natronlauge basisch gemacht und mit Chloroform extrahiert. Die Chloroformextrakte werden getrocknet und zur Trockne eingedampft, wobei ein dunkles Öl anfällt, das an einer Silicagelkolonne, mit 3%igem Methylalkohol in Chloroform als Eluiermittel gereinigt wird. Das gereinigte Öl wird in Äther gelöst und dort in sein Oxalsäuresalz, einem weißen Niederschlag überführt. Dieses Salz wird einmal aus einer Äthylalkohol-Äther-Mischung und viermal aus Äthylalkohol umkristallisiert, wobei man 2,3-Dihydro-1'-[3-(4-fluorbenzoyl)-propyl]-spiro-[benzofuran-2,3-pyrrolidin]-oxalathydrat erhält. Schmelzpunkt: 93—95°C.

Analyse:

| | | | |
|---|---|---|---|
| Berechnet für $C_{21}H_{22}FNO_2 \cdot (CO_2H)_2 . H_2O$: | 61,73% C; | 5,85% H; | 3,13% N; |
| Gefunden: | 61,96% C; | 5,48% H; | 3,02% N. |

Beispiel 3

a) Gemäß Beispiel 2a) wird eine 4-Chlor-2-fluorbenzylbromidprobe in ihr Grignardreagenz überführt, das dann mit N-Benzyl-4-piperidon zu 1-Benzyl-4-(4-chlor-2-fluorbenzyl)-4-piperidinol umgesetzt wird. Letzteres wird bei 175—180°C unter einem Druck von 0,18 mm Hg destilliert, wobei als Rückstand ein orangefarbenes Öl anfällt, das in Äther in sein Chlorwasserstoffsalz überfürt wird. Dieses Salz wird viermal aus einer Äthylalkohol-Äther-Mischung umkristallisiert und dabei gereinigt. Schmelzpunkt 211,5—213°C.

b) Zu einer Suspension aus 1,5 g Natriumhydrid als 50%ige Öldispersion in 100 ml Benzol tropft man unter Rühren bei Zimmertemperatur eine Lösung aus 8,3 g 1-Benzyl-4-(4-chlor-2-fluorbenzyl)-4-piperidinol in 75 ml Benzol. Nach beendeter Zugabe erhitzt man das Reaktionsgemisch auf Rückflußtemperatur und gibt dann 35 ml Dimethylformamid zu. Dann wird das Reaktionsgemisch nacheinander 15 Minuten unter Rückfluß erhitzt, auf Zimmertemperatur abgekühlt und tropfenweise mit insgesamt 100 ml Wasser verdünnt. Dann gibt man das Reaktionsgemisch in 1 l Eiswasser und extrahiert es dreimal mit Äther. Die vereinigten Ätherextrakte werden mit einer gesättigten Kochsalzlösung gewaschen und getrocknet. Der Äther wird unter vermindertem Druck abdestilliert, wobei ein Öl zurückbleibt, das in Hexan gekocht wird. Das Hexan wird entfernt, wobei ein gelbes Öl anfällt, das beim Stehenlassen erstarrt. Das Öl wird in Äther gelöst und dort in sein Chlorwasserstoffsalz überführt. Dieses Salz wird dreimal aus einer Athylalkohol-Äther-Mischung zu 2,3-Dihydro-1'-benzyl-6-chlorspiro-[benzofuran-2,4'-piperidin]-hydrochlorid umkristallisiert.
Schmelzpunkt: 257—259°C.

Analyse:

| | | | | |
|---|---|---|---|---|
| Berechnet für $C_{19}H_{20}ClNO \cdot HCl$: | 61,15% C; | 6,04% H; | 4,00% N; | 20,24% Cl; |
| Gefunden: | 65,01%C; | 6,07% H; | 4,05% N; | 20,11% Cl. |

c) Eine gerührte Lösung aus 6,1 g 2,3-Dihydro-1'-benzyl-6-chlorspiro-[benzofuran-2,4'-piperidin], der freien Base aus b) und 2,5 g Chlorameisensäureäthylester in 150 ml Benzol wird 18 Stunden unter Rückfluß erhitzt. Dann läßt man die Lösung auf Zimmertemperatur abkühlen, wäscht sie mit Wasser, mit gesättigter Natriumcarbonatlösung und mit gesättigter Kochsalzlösung, trocknet sie und dampft sie zur Trockne zu einem dunkeln Öl ein. Das Öl wird in ein Gemisch aus 50 ml 50%iger Kaliumhydroxidlösung und 100 ml Äthylalkohol aufgenommen, die erhaltene Mischung wird 18 Stunden unter Rückfluß erhitzt und auf Zimmertemperatur abkühlen gelassen. Dann entfernt man den Äthylalkohol unter vermindertem Druck. Die restliche wässrige Suspension wird mit Äther extrahiert und die vereinigten Atherextrakte werden mit 3n-Salzsäure gewaschen. Die angesäuerte Reaktionslösung wird mit 6n-Natronlauge basisch gemacht und mit Äther extrahiert. Die vereinigten Ätherextrakte werden getrocknet und zur Trockne eingedampft, wobei ein rohweißer Feststoff anfällt. Dieser wird in Äther gelöst und dort in sein Chlorwasserstoffsalz überführt. Nach zweimaligem Umkristallisieren dieses Salzes aus Äthylalkohol erhält man 2,3-Dihydro-6-chlorspiro-[benzofuran-2,4'-piperidin]-hydrochlorid. Schmelzpunkt: 281—288°C.

Analyse:

| | | | | |
|---|---|---|---|---|
| Berechnet für $C_{12}H_{14}ClNO \cdot HCl$: | 55,40% C; | 5,81% H; | 5.38% N; | 27,26% Cl; |
| Gefunden: | 55,21% C; | 5,86% H; | 5,38% N; | 27,07% Cl. |

d) Eine Mischung aus 2,7 g 2,3-Dihydro-5-chlorspiro-[benzofuran-2,4'-piperidin], der freien Base aus c), 1,5 g Kaliumcarbonat, einigen Kaliumjodidkörnern und 3,5 g $\gamma$-Chlor-p-fluorbutyrophenoäthylenglykolketal in 100 ml n-Butylalkohol wird 24 Stunden under Rückfluß erhitzt. Nach dem Abkühlen wird die Reaktionslösung filtriert und zu einem dunkeln Ol zur Trockne eingedampft. Dieses Öl wird in 100 ml Methylalkohol und 50 ml 3n-Salzsäure gelöst, und die erhaltene Lösung wird 18 Stunden gerührt. Dann wird das Reaktionsgemisch mit 6n-Natronlauge basisch gemacht und mit Essigsäureäthylester extrahiert. Die Essigsäureäthylesterextrakte werden mit einer Salzlauge gewaschen, getrocknet und zur Trockne eingedampft, wobei ein Öl anfällt, das bei längerem Stehenlassen zu einem lohfarbenen Feststoff erstarrt. Dieser Feststoff wird an einer Silicagelkolonne mit 2%igem Methylalkohol in Chloroform als Eluiermittel gereinigt. Der chroamtographierte Feststoff wird in Äther gelöst und dort in sein Chlorwasserstoffsalz, einem weißen Niederschlag, überführt. Nach zweimaligem Umkristallisieren dieses Salzes aus einer Äthylalkohol-Äther-Mischung erhält man 2,3-Dihydro-1'-[3-(4-fluorbenzoyl)-propyl]-6-chlorspiro-[benzofuran-2,4'-piperidin]-hydrochlorid. Schmelzpunkt: 253—255°C.

Analyse:

| | | | |
|---|---|---|---|
| Berechnet für $C_{22}H_{23}ClFNO_2 \cdot HCl$ | 62,27% C; | 5,70% H; | 3,30% N; |
| Gefunden: | 62,23% C; | 5,78% H; | 3,38% N. |

Beispiel 4

a) Gemäß Beispiel 2a) wird eine 5-Chlor-2-fluorbenzylbromidprobe in ihr Grignard Reagenz überführt, das dann mit N-Benzyl-4-piperidon zu 1-Benzyl-4-(5-chlor-2-fluorbenzyl)-4-piperidinol umgesetzt wird. Die erhaltene Verbindung wird bei 210°C unter einem Druck von 0,15 mm Hg destilliert, wobei ein orangefarbenes Öl anfällt, das in Äther gelöst wird und dort in sein Chlorwasserstoffsalz überführt wird. Nach dreimaligem Umkristallisieren dieses Salzes aus einer Äthylalkohol-Äther-Mischung erhält man 1-Benzyl-4-(5-chlor-2-fluorbenzyl)-4-piperidinol-hydrochlorid. Schmelzpunkt: 217—219°C.

b) Eine 1-Benzyl-4-(5-chlor-2-fluorbenzyl)-4-piperidinolprobe, die freie Base aus Beispiel 4a) wird gemäß Beispiel 3b zu dem Salz 2,3-Dihydro-1'-benzyl-5-chlorspiro-[benzofuran-2,4'-piperidin]-hydrochlorid, umgesetzt.

Analyse:

| | | | | |
|---|---|---|---|---|
| Berechnet für $C_{19}H_{20}ClNO \cdot HCl$: | 65,15% C; | 6,04% H; | 4,00% N; | 20,24% Cl; |
| Gefunden: | 65,21% C; | 6,11% H; | 3,98% N; | 20,06% Cl. |

c) Eine 2,3-Dihydro-1'-benzyl-5-chlorspiro-[benzofuran-2,4'-piperidin]-probe, die freie Base aus Beispiel 4b) wird gemäß Beispiel 3c) nach dreimaligem Umkristallisieren, davon zweimal aus Äthylalkohol und einmal aus einer Äthylalkohol-Äther-Mischung zu 2,3-Dihydro-5-chlorspiro-[benzofuran-2,4'-piperidin]-hydrochlorid mit einem Schmelzpunkt von 217—218°C umgesetzt.

Analyse:

| | | | | |
|---|---|---|---|---|
| Berechnet für $C_{14}H_{14}ClNO_4 \cdot HCl$: | 55,40% C; | 5,81% H; | 5,38% N; | 27,26% Cl; |
| Gefunden: | 55,53% C; | 5,84% H; | 5,40% N; | 27,11% Cl. |

d) Verfährt man mit einer Mischung aus 7,0 g 2,3-Dihydro-5-chlorspiro-[benzofuran-2,4'-piperidin], der freien Base aus Beispiel c), 10 g Kaliumcarbonat und 8,9 g $\gamma$-Chlor-p-fluorbutyrophenon-äthylenglykolketal in 250 ml n-Butylalkohol wie in Beispiel 3, so erhält man unter Rühren 2,3-Dihydro-1'-[3-(4-fluorbenzoyl)-propyl]-5-chlorspiro-[benzofuran-2,4'-piperidin]-hydrochlorid mit dem einem Schmelzpunkt von 241—243°C.

Analyse:

| | | | | |
|---|---|---|---|---|
| Berechnet für $C_{22}H_{23}ClFNO_2 \cdot HCl$: | 62,27% C; | 5,70% H; | 3,30% N; | 16,71% Cl; |
| Gefunden: | 62,46% C; | 5,72% H; | 3,32% N; | 16,91% Cl. |

Beispiel 5

a) 3 ml Acetylchlorid in 10 ml Chloroform werden unter Rühren zu einer Suspension aus 6,0 g 2,3-Dihydrospiro-[benzofuran-2,4'-piperidin] aus Beispiel 1c) und aus 14,0 g Natriumbicarbonat in 75 ml Chloroform getropft. Nach beendeter Zugabe wird die Reaktionsmischung 16 Stunden bei Zimmertemperatur gerührt, filtriert, nacheinander mit Wasser, verdünnter Salzsäure und gesättigter Kochsalzlösung gewaschen und getrocknet. Dann wird das Lösungsmittel unter vermindertem Druck abdestilliert. Der Rückstand erstarrt beim Stehenlassen. Nach zweimaligem Umkristallisieren dieses Feststoffes aus Hexan erhält man 2,3-Dihydro-1'-acetylspiro-[benzofuran-2,4'-piperidin], Schmelzpunkt: 94—97°C.

Analyse:

| | | | |
|---|---|---|---|
| Berechnet für $C_{14}H_{17}NO_2$: | 72,70% C; | 7,41% H; | 6,05% N; |
| Gefunden: | 72,85% C; | 7,47% H; | 6,08% N. |

b) Zu einer Lösung aus 4,7 g 2,3-Dihydro-1'-acetylspiro-[benzofuran-2,4'-piperidin] in 65 ml Essigsäure tropft man unter Rühren 3,8 g Salpetersäure (Dichte 1,42) in 30 ml Eisessig. Nach beendeter Zugabe erhitzt man das Reaktionsgemisch langsam 2 Stunden auf 100°C, kühlt es ab und gießt es in 500 ml Wasser. Die verdünnte Mischung wird dann mit Chloroform extrahiert. Die Chloroformextrakte werden nacheinander mit gesättigter Natriumbicarbonatlösung und gesättigter Kochsalzlösung gewaschen und getrocknet. Das Chloroform wird dann unter vermindertem Druck abdestilliert und der Rückstand mit Äther verrieben, wobei man einen orangefarbenen Niederschlag erhält, der abgetrennt wird und nach einander aus einem Athylalkohol-Ather-Gemisch und aus Athylalkohol zu 2,3-Dihydro-1'-acetyl-5-nitrospiro-[benzofuran-2,4'-piperidin] umkristallisiert wird. Schmelzpunkt: 149—150 °C

Analyse:

| | | | |
|---|---|---|---|
| Berechnet für $C_{14}H_{16}N_2O_2$: | 60,86% C; | 5,84% H; | 10,14% N; |
| Gefunden: | 60,83% C; | 5,82% H; | 10,14% N. |

c) Eine Lösung aus 3,4 g 2,3-Dihydro-1'-acetyl-5-nitrospiro-[benzofuran-2,4'-piperidin] in 150 ml 6n-Salzsäure wird 45 Stunden unter Rückfluß erhitzt und dann bei Zimmertemperatur 16 Stunden gerührt. Dann wird das Reaktionsgemisch einmal mit Ather extrahiert, mit 6n-Natronlauge basisch gemacht und dann noch dreimal mit Äther extrahiert. Die vereinigten Ätherextrakte werden getrocknet und das Lösungsmittel abdestilliert. Man erhält dabei einen gelben Rückstand, der in Äther gelöst wird und dort in sein Chlorwasserstoffsalz überführt wird. Das erhaltene Salz wird abgetrennt, getrocknet und zweimal aus Äthylalkohol zu 2,3-Dihydro-5-nitrospiro-[benzofuran-2,4'-piperidin]hydrochlorid umkristallisiert. Schmelzpunkt: 265—266°C.

Analyse:

| | | | |
|---|---|---|---|
| Berechnet für $C_{12}H_{14}N_2O_3 \cdot HCl$: | 53,24% C; | 5,59% H; | 10,35% N; |
| Gefunden: | 53,36% C; | 5,77% H; | 10,28% N. |

d) Eine Suspension aus 5,2 g 2,3-Dihydro-5-nitrospiro-[benzofuran-2,4'-piperidin], der freien Base aus c), 7,0 g Kaliumcarbonat, einigen Kaliumjodidkörnern und 6,2 g $\gamma$-Chlor-p-fluorbutyrophenon-äthylenglykolketal in 200 ml n-Butylalkohol wird 24 Stunden unter Rückfluß und unter Rühren erhitzt. Dann läßt man das Reaktionsgemisch auf Zimmertemperatur abkühlen und filtriert es. Das Filtrat wird zur Trockne eingedampft, wobei man als Rückstand ein braunes Öl erhält, das in 200 ml Methylalkohol und 100 ml 3n-Salzsäure gelöst wird. Die erhaltene Lösung wird dann bei Zimmertemperatur 18 Stunden gerührt und mit 6n-Natronlauge basisch gemacht. Die dabei erhaltene alkalische Mischung wird mit Äthylacetat extrahiert, und die Äthylacetatextrakte werden getrocknet und zur Trockne eingedampft, wobei als Rückstand ein Öl anfällt. Dieses Öl wird an einer Silicagelkolonne mit Chloroform als Eluiermittel chromatographiert, wobei man einen Feststoff erhält, der in Äther gelöst wird und dort in sein Chlorwasserstoffsalz, einen weißen Niederschlag, überführt wird. Nach zweimaligem Umkristallisieren dieses Niederschlages aus Äthylalkohol erhält man 2,3-Dihydro-1'-[3-(4-fluorbenzoyl)-propyl]-5-nitrospiro-[benzofuran-2,4'-piperidin]-hydrochlorid. Schmelzpunkt: 245—247°C.

Analyse:
Berechnet für $C_{22}H_{23}FN_2O_4 \cdot HCl$: 60,76% C; 5,56% H; 6,44% N;
Gefunden: 60,95% C; 5,61% H; 6,45% N.

Die anderen erfindungsgemäßen Verbindungen können analog zu obigen Beispielen hergestellt werden.

**Patentansprüche**

1. Verbindungen der Formel

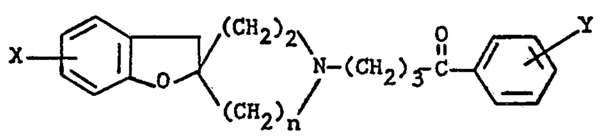

I

worin
X Wasserstoff, Nitro, Amino, Halogen, Methoxy oder Hydroxy,
Y Nitro, Amino, Halogen, Methoxy oder Hydroxy und
n 1 oder 2,
bedeuten,
sowie deren pharmazeutisch unbedenkliche Säureadditionssalze.

2. 2,3-Dihydro-1′-[3-(4-fluorbenzoyl)-propyl]-spiro-[benzofuran-2,4′-piperidin] oder sein pharmazeutisch unbedenkliches Säureadditionssalz.

3. 2,3-Dihydro-1′-[3-(4-fluorbenzoyl)-propyl]-spiro-[benzofuran-2,3′-pyrrolidin] oder sein pharmazeutisch unbedenkliches Säureadditionssalz.

4. 2,3-Dihydro-1′-[3-(4-fluorbenzoyl)-propyl]-6-chlorspiro-[benzofuran-2,4′-piperidin] oder sein pharmazeutisch unbedenkliches Säureadditionssalz.

5. 2,3-Dihydro-1′-[3-(4-fluorbenzoyl)-propyl]-5-chlorspiro-[benzofuran-2,4′-piperidin] oder sein pharmazeutisch unbedenkliches Säureadditionssalz.

6. 2,3-Dihydro-1′-[3-(4-fluorbenzoyl)-propyl]-5-nitrospiro-[benzofuran-2,4′-piperidin] oder sein pharmazeutisch unbedenkliches Säureadditionssalz.

7. Verbindungen der Formel I zur Verwendung als Arzneimittel.

8. Verfahren zur Herstellung von Verbindungen gemäß Anspruch 1, Formel I, dadurch gekennzeichnet, daß man

a) eine Verbindung der Formel

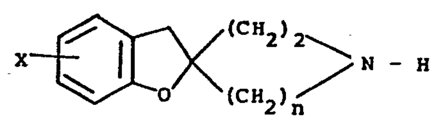

II

worin X und n die in Formel I in Anspruch 1 angegebene Bedeutung haben, mit einem $\gamma$-Chlorbutyrophenonäthylenglykolketal der Formel

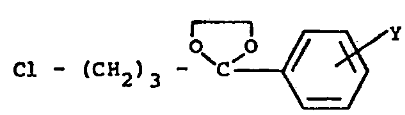

III

worin Y die in Formel I in Anspruch 1 angegebene Bedeutung hat, in einem Lösungsmittel zu einer Verbindung der Formel

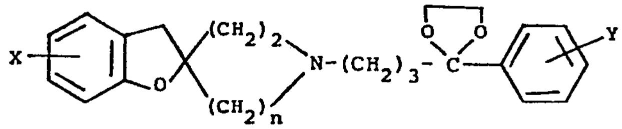

IV

umsetzt,

b) die erhaltene Verbindung hydrolysiert und

c) gegebenenfalls eine Verbindung der Formel I, worin X und/oder Y Methoxy bedeuten, nach üblichen Methoden zu einer Verbindung der Formel I, worin X und/oder Y jeweils Hydroxy bedeuten, demethyliert.

9. Verfahren gemäß Anspruch 8, dadurch gekennzeichnet, daß die Reaktion mit $\gamma$-Chlorbutyrophenon bei einer Temperatur von 25°C bis Rückflußtemperatur des Reaktionsgemisches in Gegenwart eines Säurefängers erfolgt.

10. Verfahren gemäß Anspruch 8, dadurch gekennzeichnet, daß die Hydrolyse mit einer starken Säure in einem Lösungsmittel bei Zimmertemperatur durchgeführt wird.

11. Verfahren gemäß Anspruch 8, dadurch gekennzeichnet, daß die Demethylierung mit einer auf Rückflußtemperatur erhitzten Bromwasserstoffsäure durchgeführt wird.

**Claims**

1. A compound of the formula

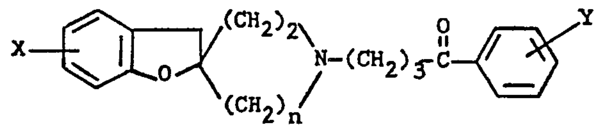

I

or a pharmaceutically acceptable acid addition salt thereof in which X is hydrogen, nitro, amino, halogen, methoxy or hydroxy; Y is nitro, amino, halogen, methoxy or hydroxy; and n is the integer 1 or 2.

2. The compound defined in claim 1 which is 2,3-dihydro-1′-[3-(4-fluorobenzoyl)propyl]spiro[benzofuran-2,4′-piperidine] or a pharmaceutically acceptable acid addition salt thereof.

3. The compound defined in claim 1 which is 2,3-dihydro-1′-[3-(4-fluorobenzoyl)propyl]spiro[benzofuran-2,3′-pyrrolidine] or a pharmaceutically acceptable acid addition salt thereof.

4. The compound defined in claim 1 which is 2,3-dihydro-1′-[3-(4-fluorobenzoyl)propyl]-6-chlorospiro[benzofuran-2,4′-piperidine] or a pharmaceutically acceptable acid addition salt thereof.

5. The compound defined in claim 1 which is 2,3-dihydro-1′-[3-(4-fluorobenzoyl)propyl]-5-chlorospiro[benzofuran-2,4′-piperidine] or a pharmaceutically acceptable acid addition salt thereof.

6. The compound defined in claim 1 which is 2,3-dihydro-1′-[3-(4-fluorobenzoyl)propyl]-5-nitrospiro[benzofuran-2,4′-piperidine] or a pharmaceutically acceptable acid addition salt thereof.

7. A pharmaceutical composition comprising between 0.5 and 70% by weight of a compound defined in claim 1 and a pharmaceutically acceptable carrier thereof.

8. A method for preparing a compound defined in claim 1 which comprises

a) reacting a compound of the formula

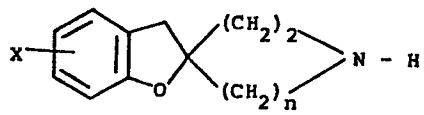

II

in which X and n are as defined in formula I in claim 1 with a $\gamma$-chlorobutyrophenone ethylene glycol ketal of the formula

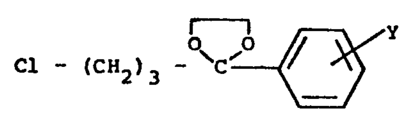

III

in which Y is as defined in formula I in a solvent to provide to a compound of the formula

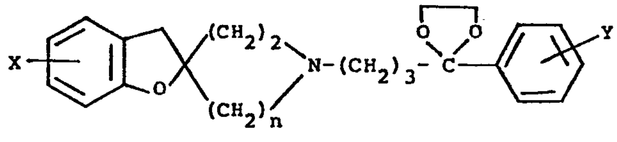

IV

b) subjecting this compound to hydrolysis and

c) optionally demethylating a compound of the formula I in which X or Y or both are methoxy by any convenient method known in the art to produce the corresponding compound of the formula I in which X or Y both are hydroxy.

9. The method according to claim 8 in which the reaction with γ-chlorobutyrophenone is carried out at a temperature of from 25°C to reflux of the reaction mixture in the presence of an acid scavenger.

10. The method according to claim 8 in which the hydrolysis is effected with a strong acid in a solvent at ambient temperature.

11. The method according to claim 8 in which the demethylation is carried out with a refluxing hydrobromic acid.

**Revendications**

1. Composés de formule

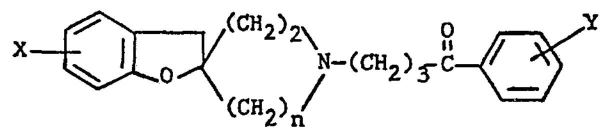

I

dans laquelle

X représente l'hydrogène, un groupe nitro ou amino, un halogène ou un groupe méthoxy ou hydroxy;

Y représente un groupe nitro ou amino, un halogène ou un groupe méthoxy ou hydroxy, et

n est 1 ou 2,

et leurs sels d'addition avec des acides pharmaceutiquement acceptables.

2. 2,3-dihydro-1′-[3-(4-fluorobenzoyl)-propyl]-spiro-[benzofuranne-2,4′-pipéridine] ou un sel d'addition avec un acide pharmaceutiquement acceptable de celle-ci.

3. 2,3-dihydro-1′-[3-(4-fluorobenzoyl)-propyl]-spiro-[benzofuranne-2,3′-pyrrolidine] ou un sel d'addition avec un acide pharmaceutiquement acceptable de celle-ci.

4. 2,3-dihydro-1′-[3-(4-fluorobenzoyl)-propyl]-6-chlorospiro-[benzofuranne-2,4′-pipéridine] ou un sel d'addition avec un acide pharmaceutiquement acceptable de celle-ci.

5. 2,3-dihydro-1′-[3-(4-fluorobenzoyl)-propyl]-5-chlorospiro-[benzofuranne-2,4′-pipéridine] ou un sel d'addition avec un acide pharmaceutiquement acceptable de celle-ci.

6. 2,3-dihydro-1′-[3-(4-fluorobenzoyl)-propyl]-5-nitrospiro-[benzofuranne-2,4′-pipéridine] ou un sel d'addition avec un acide pharmaceutiquement acceptable de celle-ci.

7. Composés de formule I pour utilisation comme médicaments.

8. Procédé de préparation de composés de formule I selon la revendication 1, caractérisés en ce que:

a) on fait réagir un composé de formule

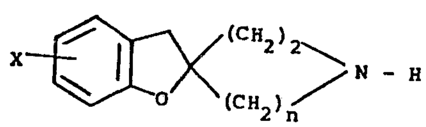

II

dans laquelle X et n ont les significations indiquées pour la formule I dans la revendication 1, avec un éthylène glycol cétal de γ-chlorobutyrophénone de formule

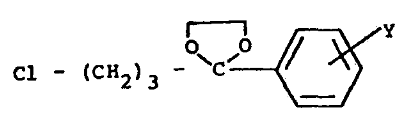

III

dans laquelle Y a la signification indiquée pour la formule I dans la revendication 1, dans un solvant, pour obtenir un composé de formule

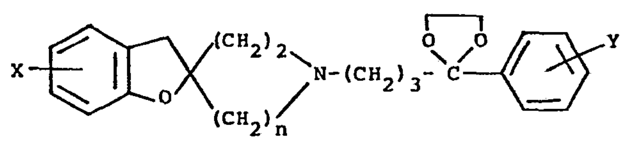

IV

b) on hydrolyse le composé obtenu, et

c) éventuellement on déméthyle un composé de formule I, où X et/ou Y représentent un groupe méthoxy, selon des procédés usuels, pour obtenir un composé de formule I où X et/ou Y représentent chacun un groupe hydroxy.

9. Procédé selon la revendication 8, caractérisé en ce que la réaction avec la $\gamma$-chlorobutyrophénone est conduite à une température de 25°C à la température de reflux du mélange réactionnel, en présence d'un accepteur d'acide.

10. Procédé selon la revendication 8, caractérisé en ce que l'hydrolyse est effectuée avec un acide fort dans un solvant à la température ambiante.

11. Procédé selon la revendication 8, caractérisé en ce que la déméthylation est effectuée avec un acide bromhydrique chauffé à température de reflux.